# EUROPÄISCHE PATENTSCHRIFT

(11) **EP 2 256 155 B1**
(45) Veröffentlichungstag und Bekanntmachung des Hinweises auf die Patenterteilung: **03.05.2017**
(21) Anmeldenummer: 10179005.3
(22) Anmeldetag: 07.06.2004
(51) Int. Cl.: C08J 9/26, A61L 27/26, A61L 27/38, A61L 27/56, A61L 27/58

(54) **MATRIX, ZELLIMPLANTAT, VERFAHREN ZU DEREN HERSTELLUNG UND DEREN VERWENDUNG**
MATRIX, CELL IMPLANTATION AND METHOD FOR THEIR PRODUCTION AND USE
MATRICE, IMPLANT CELLULAIRE, LEUR PROCÉDÉ DE PRODUCTION ET LEUR UTILISATION

(30) Priorität: 06.06.2003 DE 10325807
(43) Veröffentlichungstag der Anmeldung: 01.12.2010
(62) Teilanmeldung aus: 04739671.8
(73) Patentinhaber: Human Auto Cell Limited, Hersham Surrey KT12 4BA (GB)
(72) Erfinder: Görne, Martin, 22337 Hamburg (DE); Kaufmann, Peter-Matthias, 30900 Wedemark (DE)
(74) Vertreter: Reitstötter Kinzebach

(56) Entgegenhaltungen:
- WO-A-98/44027
- WO-A-99/09149
- WO-A-99/32204
- WO-A-03/064509
- WO-A1-94/25079
- WO-A2-01/35932
- WO-A2-2004/031371
- US-A- 5 514 378
- US-A- 5 626 861
- US-B1- 6 337 198
- HOU Q ET AL: "Porous polymeric structures for tissue engineering prepared by a coagulation, compression moulding and salt leaching technique" BIOMATERIALS, ELSEVIER SCIENCE PUBLISHERS BV., BARKING, GB, Bd. 24, Nr. 11, Mai 2003 (2003-05), Seiten 1937-1947, XP004412414 ISSN: 0142-9612
- SOHIER J ET AL: "A novel method to obtain protein release from porous polymer scaffolds: emulsion coating" JOURNAL OF CONTROLLED RELEASE, ELSEVIER SCIENCE PUBLISHERS B.V. AMSTERDAM, NL, Bd. 87, Nr. 1-3, 21. Februar 2003 (2003-02-21), Seiten 57-68, XP004412747 ISSN: 0168-3659
- CHEN G ET AL: "Preparation of poly(l-lactic acid) and poly(dl-lactic-co-glycolic acid) foams by use of ice microparticulates" BIOMATERIALS, ELSEVIER SCIENCE PUBLISHERS BV., BARKING, GB, Bd. 22, Nr. 18, September 2001 (2001-09), Seiten 2563-2567, XP004273584 ISSN: 0142-9612
- HARRIS L D ET AL: "OPEN PORE BIODEGRADABLE MATRICES FORMED WITH GAS FOAMING" JOURNAL OF BIOMEDICAL MATERIALS RESEARCH, WILEY, NEW YORK, NY, US, Bd. 42, 1998, Seiten 396-402, XP001021110 ISSN: 0021-9304
- WHANG K ET AL: "A novel method to fabricate bioabsorbable scaffolds" POLYMER, ELSEVIER SCIENCE PUBLISHERS B.V, GB, Bd. 36, Nr. 4, 1. Februar 1995 (1995-02-01), Seiten 837-842, XP004025968 ISSN: 0032-3861

## Beschreibung

Die vorliegende Erfindung betrifft autologe Implantate zur Verwendung bei der Behandlung eines Wesens, das einer Leber- oder Pankreastransplantation bedarf.

Tissue engineering ist ein interdisziplinäres Gebiet, das Ingenieur- und Materialwissenschaften mit der Medizin verbindet. Ziel ist es, geschädigtes Gewebe wiederherzustellen oder seine Funktion zu verbessern.

Das Prinzip des Tissue engineering ist denkbar einfach: Zunächst werden dem Patienten einige Zellen entnommen und *in vitro* vermehrt. Die vermehrten Zellen können dann in eine Gerüstsubstanz eingebettet werden, wodurch ein kompletter, lebender Gewebeersatz entsteht, der dem Patienten wieder transplantiert wird. Im Gegensatz zur herkömmlichen, einen geeigneten Spender voraussetzenden und in der Regel eine lebenslängliche medikamentöse Immunsuppression erfordernden allogenen Transplantation bietet dieses Verfahren den entscheidenden Vorteil, körpereigene (autologe) Zellen verwenden zu können.

Von besonderer Bedeutung für die Annahme und Funktionsfähigkeit der Implantate sind Art und Aufbau der verwendeten Gerüstsubstanz, im folgenden auch Matrix genannt. Abgesehen von dem zu verwendenden Material, nämlich in der Regel biologisch abbaubaren Polymeren, spielen Porengröße, Porosität und Oberfläche genauso wie die Porengestalt, die Morphologie der Porenwand und die Konnektivität zwischen den Poren eine entscheidende Rolle für die weitere Entwicklung der in der Gerüstsubstanz eingebetteten Zellen und letztendlich für den dreidimensionalen Aufbau des zu regenerierenden Gewebes oder Organs.

Verfahren zur Erzeugung derartiger Biomatrices sind bereits bekannt. So wurden bereits Techniken aus dem Textilbereich angewendet, um webartige und auch vliesartige, faserige Biomatrices herzustellen. Ein weiteres geläufiges Verfahren, bei dem Salzkristalle zunächst in das biologisch abbaubare Polymer eingearbeitet und anschließend wieder herausgelöst werden, ermöglicht es, die Porengröße über die Größe der Salzpartikel und die Porosität über das Salz/Polymerverhältnis zu kontrollieren (WO 98/44027). Bei einer Abwandlung des Verfahrens werden die in einem Lösungsmittel gelösten, biologisch abbaubaren Polymere auf ein sogenanntes porogenes Material aufgetragen, das anschließend aus dem Verbundmaterial wieder herausgelöst wird, wodurch Poren mit der Gestalt des negativen Abbildes besagten porogenen Materials hinterlassen werden (WO 01/87575 A2). Auch beschichtete Matrices sind bereits bekannt (siehe z.B. WO 99/09149 A1). Hou und Mitarbeiter beschreiben in Biomaterials, Bd. 24, Seiten 1937-1947 eine Reihe verschiedener Verfahren zur Herstellung polymerer Matrices. WO 01/35932 beschreibt poröse Matrices auf Basis eines biologisch verträglichen Polymers und Verfahren zu deren Herstellung. WO 98/44027 beschreibt ein Verfahren zur Herstellung offenporiger, biologisch abbaubarer Matrices. US 6337198 B1 beschreibt bioabbaubare und biokompatible poröse Matrices mit einer im Wesentlichen kontinuierlichen Polymerphase. WO 2004/031371 A2 beschreibt Polymermatrices, die für Tissue engineering verwendet werden können. WO 94/25079 A1 beschreibt poröse, bioabbaubare Polymere für Zelltransplantationen.

Nichtsdestotrotz stellen die bisher mit diesem Verfahren erzeugten Biomatrices insbesondere im Hinblick auf die Annahme und Funktionsfähigkeit darauf aufbauender Implantate nicht in allen Fällen zufrieden. Insbesondere mit Leber- und Pankreasimplantaten ist bisher noch kein annehmbarer Ersatz der Organe gelungen.

Die der vorliegenden Erfindung zugrunde liegende Aufgabe, ein solches funktionsfähiges Implantat zur Verfügung zu stellen, löst die Erfindung durch bestimmte Biomatrices und entsprechende Implantate, die mit einem speziellen Verfahren erhältlich sind.

Gegenstand der vorliegenden Erfindung sind daher die in den Patentansprüchen definierten Gegenstände.

Der Porositätsgrad ist die zahlenmäßige Angabe in % zum Anteil des Porenvolumens am Gesamtvolumen der Matrix.

Mit Poren bezeichnet man die in der porösen Matrix vorhandenen Hohlräume, die im vorliegenden Fall im 2-dimensionalen Schnitt eine eckige, insbesondere oktogonale bzw. 3-dimensional gesehen eine kantige Gestalt haben. Vorzugsweise ist die Gestalt des weiteren durch Ausziehungen gekennzeichnet, so dass man die Gestalt der Hohlräume mit der Form von Nervenzellen vergleichen kann. Die Größe einer Pore kann mit Hilfe eines Durchmessers angegeben werden, das heißt dem Mittel aus dem längsten und dem kürzesten Durchmesser der im 2-dimensionalen Schnitt erkennbaren Poren.

Eine poröse Matrix weist Poren mit unterschiedlichen Größen auf, wobei die Größen über einen bestimmten Bereich verteilt sind (Porengrößenverteilung). Erfindungsgemäß von Bedeutung ist, dass eine Matrix eine breite Porengrößenverteilung aufweist. Von besonderem Vorteil sind Matrices, die Poren mit einer Größe von 130 µm oder weniger aufweisen.Von besonderem Vorteil sind Matrices, die Poren mit einer Größe von 370 µm oder mehr aufweisen. Matrices, die sowohl Poren mit einer Größe 130 µm oder weniger als auch Poren mit einer Größe von 370 µm oder mehr aufweisen, gehören zur Erfindung. Diese Werte lassen sich beliebig zu Mindestbereichen, über die sich die Porengrößenverteilung erstrecken soll, kombinieren, wobei insbesondere die Bereiche 150 bis 300, 140 bis 350 und 130 bis 370 µm zu nennen sind. Insbesondere bevorzugt ist es, wenn die jeweilige Porengrößenverteilung Häufigkeitsmaxima außerhalb des Bereichs von 150 bis 300 µm aufweist, d.h. ein Häufigkeitsmaximum oberhalb einer Porengröße von 300 µm und ein weiteres Häufigkeitsmaximum unterhalb einer Porengröße von 150 µm liegt.

Eine typische poröse Matrix weist folgende Porengrößenverteilung auf. 0,5 % bis 6 %, vorzugsweise 1 % bis 5 %, noch bevorzugter 2 % bis 4 % und insbesondere 3 % Poren mit einem mittleren Durchmesser im Bereich von 70 bis 100 um; 2 % bis 8 %, vorzugsweise 3 % bis 7 %, noch bevorzugter 4 % bis 6 % und insbesondere etwa 5 % Poren mit einem mittleren Durchmesser im Bereich von 101 bis 115 um; 2 % bis 8 %, vorzugsweise 3 % bis 7 %, noch bevorzugter 4 % bis 6 % und insbesondere 5 % Poren mit einem mittleren Durchmesser im Bereich von 116 bis 130 um; 1 % bis 7 %, vorzugsweise 2 % bis 6 %, noch bevorzugter 3 % bis 5 % und insbesondere 4 % Poren mit einem mittleren Durchmesser im Bereich von 131 bis 300 um; 11 % bis 23 %, vorzugsweise 13 % bis 21 %, noch bevorzugter 15 % bis 19 % und insbesondere 17 % Poren mit einem mittleren Durchmesser im Bereich von 301 bis 330 um; 4 % bis 10 %, vorzugsweise 5 % bis 9 %, noch bevorzugter 6 % bis 8 % und insbesondere 7 % Poren mit einem mittleren Durchmesser im Bereich von 331 bis 360 um; 5 % bis 17 %, vorzugsweise 7 % bis 15 %, noch bevorzugter 9 % bis 13 % und insbesondere 11 % Poren mit einem mittleren Durchmesser im Bereich von 361 bis 390 um; 7 % bis 19 %, vorzugsweise 9 % bis 17 %, noch bevorzugter 11 % bis 15 % und insbesondere 13 % Poren mit einem mittleren Durchmesser im Bereich von 391 bis 420 um; 3 % bis 9 %, vorzugsweise 4 % bis 8 %, noch bevorzugter 5 % bis 7 % und insbesondere 6 % Poren mit einem mittleren Durchmesser im Bereich von 421 bis 450 um; 12 % bis 24 %, vorzugsweise 14 % bis 22 %, noch bevorzugter 16 % bis 20 % und insbesondere 18 % Poren mit einem mittleren Durchmesser im Bereich von 451 bis 480 um; und 5 % bis 17 %, vorzugsweise 7 % bis 15%, noch bevorzugter 9 % bis 13 % und insbesondere 11 % Poren mit einem mittleren Durchmesser im Bereich von 481 bis 510 µm. Es ergibt sich also in der Regel eine Porengrößenverteilung mit mehr als einem Maximum, was einer Häufung von Poren in mehr als einem Größenbereich entspricht. Dies ist für die Eigenschaften poröser Matrices von besonderer Bedeutung.

Das Hohlraumvolumen und damit der Porositätsgrad sind in an sich bekannter Weise durch Porosimetrie zu bestimmen.

Die Porengrößen und damit auch die Porengrößenverteilung können beispielsweise durch Rasterelektronenmikroskopie bestimmt werden. Dazu werden dünne Schnitte der zu untersuchenden Matrix angefertigt und mit Gold überzogen. Die rasterelektronenmikroskopischen Aufnahmen werden ausgewertet, indem man sämtliche Poren einer definierten Fläche ausmisst, d.h. für jede Pore den längsten und den kürzesten Durchmesser bestimmt, aus beiden Werten die Summe bildet und die Summe durch 2 dividiert.

Der Begriff "Matrix" bezieht sich auf einen dreidimensionalen Träger, der für die Ansiedlung von Zellen geeignet ist. In diesem Sinne dient die Matrix als dreidimensionale Strukturvorlage (Templat) für die Ansiedlung von Zellen bzw. Geweben. Diese Ansiedlung kann in vitro oder in vivo erfolgen. Ferner dient die Matrix bei Transplantationen zur Lokalisierung des Transplantats und auch als Platzhalter für Gewebe, das sich nach und nach in vivo bildet.

Das Polymer kann im Prinzip jedes im Bereich der Medizin und insbesondere der Transplantationsmedizin verwendbare Polymer sein. Biologisch verträglich sind demnach auch Polymere, die von einem Wirt zwar als fremd erkannt werden, deren Abstoßung sich aber durch entsprechende Immunsuppression unterdrücken lässt. Es können Polymere zum Einsatz kommen, die im Wesentlichen biologisch nicht abbaubar sind. Bevorzugt sind allerdings Polymere, die zumindest zum überwiegenden Teil biologisch abbaubar sind.

Der Ausdruck "biologisch abbaubar" bezieht sich auf ein Material, das Lebewesen (oder von Lebewesen ableitbare Körperflüssigkeiten oder Zellkulturen) in verstoffwechselbare Produkte zu überführen vermögen. Zu biologisch abbaubaren Polymeren gehören beispielsweise bioresorbierbare und/oder bioerodierbare Polymere. Bioerodierbar bezeichnet die Fähigkeit, in biologischen Flüssigkeiten lösbar oder suspendierbar zu sein. Bioresorbierbar meint die Fähigkeit, von Zellen, Gewebe oder Flüssigkeiten eines Lebewesens aufgenommen werden zu können.

Zu erfindungsgemäß geeigneten biologisch abbaubaren Polymeren gehören im Prinzip sämtliche im Bereich der Medizin verwendbaren Polymere, wozu neben den im Bereich des Tissue engineering bereits etablierten Polymeren beispielsweise auch Polymere gehören, die in Wirkstoffabgabevorrichtungen, wie Pflastern und Wirkstoffimplantaten, Eingang gefunden haben.

Zu geeigneten natürlichen Polymeren gehören beispielsweise Polypeptide wie Albumin, Fibrinogen, Collagen und Gelatine, sowie Polysaccharide, wie Chitin, Chitosan, Alginat und Agarose. Unter Umständen können diese natürlichen Polymere auch modifiziert sein, beispielsweise können Proteine wie Collagen quervernetzt sein.

Zu geeigneten synthetischen Polymeren gehören beispielsweise bestimmte Polyanhydride, insbesondere Poly(sebacinsäure-hexadecandisäure), Poly(ε-caprolacton), Poly(orthoester), und vor allem Poly(a-hydroxyester) wie Poly(glykolsäure), Poly(milchsäure) und Poly(glykolsäuremilchsäure). So basieren die erfindungsgemäßen Implantate vorzugsweise auf biologisch abbaubaren Polymeren, die Wiederholungseinheiten der Formel (I) enthalten: 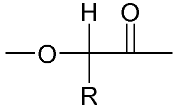 worin R¹ für Wasserstoff oder Methyl steht. Was die Milchsäureeinheiten angeht, so wird die L-Form (das S-Enantiomer) bevorzugt. Als besonders bevorzugtes Polymer ist Poly(glykolsäuremilchsäure) mit einem Glykolsäure zu Milchsäure-Verhältnis von 99:1 bis 1:99, vorzugsweise 10:90 bis 90:10, beispielsweise 15:85 mol-% zu nennen.

Gemische aus zwei oder mehreren Polymeren können ebenfalls zweckmäßig sein.

Neben der Art des Polymers bestimmt auch sein Molekulargewicht die Eigenschaften der resultierenden Matrix mit. Allgemein gilt, dass die Porosität der Matrix mit zunehmendem Molekulargewicht des verwendeten Polymers abnimmt. Dies gilt insbesondere dann, wenn bei der Herstellung der Matrix das Material geschäumt wird, d.h. unter Druck mit einem Gas wie CO₂ versetzt wird, das sich zunächst in dem Polymer löst und bei Absenkung des Drucks Poren bildet.

Ferner wirkt sich die Kristallinität des eingesetzten Polymers auf die Eigenschaften der resultierenden Matrix aus. Hier gilt, dass die Porosität der resultierenden Matrix mit abnehmender Kristallinität im allgemeinen zunimmt, weshalb amorphes Polymer insbesondere für Matrices mit hoher Porosität bevorzugt ist. Auch dieser Aspekt spielt insbesondere dann eine Rolle, wenn das Material bei der Herstellung der Matrix geschäumt wird.

Ein weiterer Gegenstand sind poröse Matrices auf Basis eines biologisch abbaubaren Polymers, die dadurch gekennzeichnet sind, dass die Oberfläche der Matrix mit wenigstens einem extrazellulären Matrixprotein beschichtet ist.

Extrazelluläre Matrixproteine sind allgemein bekannt. Bevorzugt sind Collagene, insbesonders Collagene des Typs I und IV, Laminin und Fibronectin. Diese Proteine können in an sich bekannter Weise in aufgereinigter Form hergestellt oder auch kommerziell erworben werden. Gemäß einer Ausführungsform enthalten Beschichtungen poröser Matrices als extrazelluläres Matrixprotein Fibronectin. Gemäß einer weiteren Ausführungsform enthalten Beschichtungen poröser Matrices als extrazelluläres Matrixprotein ein Gemisch aus Collagen vom Typ I, Laminin und Collagen vom Typ IV, wobei es in diesem Fall bevorzugt ist, dass das Gemisch die Proteine in etwa gleichen Gewichtsanteilen enthält.

Besonders bevorzugt sind Matrices, die in der oben beschriebenen Art und Weise beschichtet sind und die wenigstens eines der folgenden zusätzlichen Kriterien erfüllen:
- Die Poren der Matrices weisen die oben angegebenen Porengrößen bzw. Porengrößenverteilung auf;
- der Porositätsgrad beträgt 93 bis 98 %;
- die Poren weisen die oben angegebene Gestalt auf:
- das biologisch abbaubare Polymer ist eines der oben angegebenen natürlichen oder synthetischen Polymere, insbesondere Poly(glycolsäure-milchsäure) mit einem Milchsäureanteil von 85 mol-% und einem Glykolsäureanteil von 15 mol-%.

Derart beschichtete Matrices sind beispielsweise dadurch erhältlich, dass man die unbeschichtete Matrix in eine Lösung taucht, die das für die Beschichtung vorgesehene Protein oder Proteingemisch enthält, und anschließend die mit der Lösung befeuchtete Matrix trocknet. Dabei ist es in der Regel so, dass in Abhängigkeit von den Abmessungen des zu beschichtenden Matrixkörpers die Lösung vor allem die äußeren Bereiche des Matrixkörpers benetzt, während in das Innere des Matrixkörpers vergleichsweise weniger Lösung vordringt. Dies kann zur Folge haben, dass eine gleichmäßige Beschichtung der gesamten Matrixoberfläche nicht resultiert, sondern die Beschichtungsdichte von außen nach innen abnimmt.

Alternativ oder zusätzlich zu einer Beschichtung können biologisch aktive Substanzen im Polymer aufgenommen oder sogar damit verknüpft sein. Hierzu gehören beispielsweise synthetische Wirkstoffe (anorganische oder organische Moleküle), Proteine, Polysaccharide und weitere Zucker, Lipide und Nukleinsäuren, welche z.B. das Zellwachstum, die Zellmigration, die Zellteilung, die Zelldifferenzierung und/oder das Gewebewachstum beeinflussen, bzw. therapeutische, prophylaktische oder diagnostische Wirkungen besitzen. Zu nennen sind beispielsweise gefäßaktive Wirkstoffe, neuroaktive Wirkstoffe, Hormone, Wachstumsfaktoren, Cytokine, Steroide, Antikoagulanzien, entzündungshemmende Wirkstoffe, immunmodulierende Wirkstoffe, zytotoxische Wirkstoffe, Antibiotika und antivirale Wirkstoffe.

Die vorliegende Erfindung betrifft auch ein Verfahren zur Herstellung einer porösen Matrix auf Basis eines biologisch verträglichen Polymers oder Polymergemisches, das dadurch gekennzeichnet ist, dass man ein Gemisch aus Polymerpartikeln und Kochsalzpartikeln mit definierter Korngröße kompaktiert und anschließend das Kochsalz herauslöst.

Polymerpartikel mit einer Korngröße im Bereich von 20 bis 950 µm, vorteilhafterweise im Bereich von 20 bis 760 µm und insbesondere im Bereich von 108 bis 250 µm und Kochsalzpartikel mit einer Korngröße im Bereich von 90 bis 670 µm, vorteilhafterweise im Bereich von 110 bis 520 µm und insbesondere im Bereich von 250 bis 425 µm haben sich zur Einstellung der gewünschten Porengrößen bzw. Porengrößenverteilung als zweckmäßig erwiesen. Ferner hat sich ein Gewichtsverhältnis von Polymerpartikeln zu Kochsalzpartikeln im Bereich von 1:100 bis 1:10, vorteilhafterweise im Bereich von 1:50 bis 1:15 und insbesondere im Bereich von 1:20 bis 1:18 zur Einstellung der gewünschten Porosität als zweckmäßig erwiesen.

Es hat sich weiterhin als zweckmäßig erwiesen, Salz und Polymer mit einer bestimmten Korngrößenverteilung zu verwenden. Was das zur Herstellung der Matrix verwendete Kochsalz angeht, so ist es günstig, wenn der Anteil an Salz mit einer Korngröße von 250 µm bis 320 µm 15 % bis 50 %, vorteilhafterweise 18 % bis 42 % und bevorzugterweise 22 % bis 28 %; der Anteil an Salz mit einer Korngröße von 330 µm bis 380 µm 20 % bis 65 %, vorteilhafterweise 30 % bis 52 % und bevorzugterweise 42 % bis 46 %; und der Anteil an Salz mit einer Korngröße von 390 µm bis 425 µm 15 % bis 62 %, vorteilhafterweise 25 % bis 42 %, und bevorzugterweise 29 % bis 33 % beträgt, wobei sich die Prozentangaben auf das zur Herstellung insgesamt verwendete Gewicht an Salz beziehen. Anteile mit Korngrößen ober- und/oder unterhalb der angegebenen Bereiche sind damit nicht ausgeschlossen.

Einer speziellen Ausführungsform zufolge hat es sich als günstig erwiesen, wenn der Anteil an Kochsalzpartikeln mit einer Korngröße von 108 µm bis 140 µm 1 bis 15 Gew.-%, vorzugsweise 4 bis 12 Gew.-% und insbesondere 7 bis 9 Gew.-%, der Anteil an Salz mit einer Korngröße von 145 µm bis 180 µm 1 bis 11 Gew.-%, vorzugsweise 3 bis 9 Gew.-% und insbesondere 5 bis 7 Gew.-%, der Anteil an Salz mit einer Korngröße von 185 µm bis 220 µm 3 bis 21 Gew.-%, vorzugsweise 7 bis 17 Gew.-% und insbesondere 10 bis 14 Gew.-%, der Anteil an Salz mit einer Korngröße von 225 µm bis 250 µm 1 bis 11 Gew.-%, vorzugsweise 3 bis 9 Gew.-% und insbesondere 5 bis 7 Gew.-%, der Anteil an Salz mit einer Korngröße von 250 µm bis 320 µm 15 bis 50 Gew.-%, vorzugsweise 18 bis 42 Gew.-% und insbesondere 22 bis 28 Gew.-%, der Anteil an Salz mit einer Korngröße von 330 µm bis 380 µm 15 bis 50 Gew.-%, vorzugsweise 18 bis 42 Gew.-% und insbesondere 22 bis 28 Gew.-%, und der Anteil an Salz mit einer Korngröße von 390 µm bis 425 µm 5 bis 29 Gew.-%, vorzugsweise 10 bis 24 Gew.-% und insbesondere 15 bis 19 Gew.-% beträgt.

Was das zur Herstellung der Matrix verwendete Polymer angeht, so ist es günstig, wenn der Anteil an Polymer mit einer Korngröße von 108 µm bis 140 µm 5 % bis 50 %, vorteilhafterweise 10 % bis 30 % und bevorzugterweise 14 % bis 18 %; der Anteil an Polymer mit einer Korngröße von 145 µm bis 180 µm 10 % bis 55 %, vorteilhafterweise 15 % bis 40 % und bevorzugterweise 20 % bis 24; der Anteil an Polymer mit einer Korngröße von 185 µm bis 220 µm 18 % bis 88 %, vorteilhafterweise 32 % bis 76 % und bevorzugterweise 43 % bis 49 %, und der Anteil an Polymer mit einer Korngröße von 225 µm bis 250 µm 5 % bis 45 %, vorteilhafterweise 10 % bis 28 % und bevorzugterweise 14 % bis 18 % beträgt, wobei sich die Prozentangaben auf das zur Herstellung insgesamt verwendete Gewicht an Polymer beziehen.

Um Salz- bzw. Polymerpartikel der gewünschten Korngrößenverteilung zu erhalten, ist es in der Regel zweckmäßig, handelsübliche Ware zunächst zu zerkleinern. Dies kann in dafür gebräuchlichen Vorrichtungen, z.B. Schlagwerken oder Mühlen, erfolgen. Bestimmend für die gewünschte Korngrößenverteilung ist allerdings das anschließende Aussieben mit Hilfe gebräuchlicher Analysesiebe.

Das Kompaktieren erfolgt vorzugsweise durch Einwirkung von Druck. Dazu kann man das Polymer/Kochsalz-Gemisch in einer herkömmlichen Hydraulikpresse bei einem Stempeldruck im Bereich von etwa 780 psi bis 1450 psi, vorteilhafterweise im Bereich von etwa 840 psi bis 1230 psi und insbesondere im Bereich von etwa 900 psi bis 1100 psi verpressen. Es hat sich als zweckmäßig erwiesen, den Druck etwa 10 s bis 360 s, vorteilhafterweise etwa 40 s bis 180 s und insbesondere etwa 50 s bis 70 s bei Temperaturen im Bereich von 18 °C bis 25 °C einwirken zu lassen.

Das Herauslösen des Kochsalzes gelingt beispielsweise mit Wasser oder wässrigen Lösungen. So kann man das kompaktierte Gemisch (Matrixrohling) etwa 1 h bis 80 h, vorteilhafterweise etwa 12 h bis 62 h und insbesondere etwa 36 h bis 60 h wässern.

Zudem ist es von Vorteil, wenn das kompaktierte Gemisch vor dem Herauslösen des Kochsalzes zunächst in einer CO₂-Atmosphäre gelagert wird. So kann man das kompaktierte Gemisch beispielsweise bei einem CO₂-Druck im Bereich von etwa 140 psi bis 1650 psi, vorteilhafterweise im Bereich von etwa 360 psi bis 1120 psi und insbesondere im Bereich von etwa 800 psi bis 900 psi begasen, wobei sich hierbei Zeiten im Bereich von etwa 1 h bis 180 h, vorteilhafterweise im Bereich von etwa 3 h bis 60 h und insbesondere im Bereich von etwa 12 h bis 36 h als zweckmäßig erwiesen haben. Danach verringert man den Druck, wobei die Druckabsenkungsgeschwindigkeit Einfluss auf die Porenbildung hat. Wenngleich die Verwendung von CO₂ bevorzugt ist, können andere Gase, wie Luft, Stickstoff, Helium, Neon, Krypton, Argon, Xenon oder Sauerstoff ebenfalls geeignet sein.

Anschließend wird das Wasser oder die wässrige Lösung zwecks Trocknung in an sich bekannter Weise entfernt. Dazu kann man die Matrix beispielsweise auf Saugpapier legen.

Einer bevorzugten Ausführungsform zufolge setzt man dem Gemisch aus Polymerpartikeln und Kochsalzpartikeln eine Polymerlösung zu und entfernt das Lösungsmittel, bevor man kompaktiert. Dabei können Polymerpartikel und Polymerlösung auf dem gleichen Polymer basieren. Es kann sich aber auch um unterschiedliche Polymere, insbesondere mit unterschiedlicher biologischer Abbaubarkeit handeln. Der von Polymerlösung hat den Vorteil, dass quasi Stützpfeiler in die Matrix eingezogen werden, wodurch sich die mechanischen Eigenschaften der Matrix verbessern lassen. Eine solche Matrix weist insbesondere eine geringere Neigung auf zu zerkrümeln.

Das verwendete Lösungsmittel sollte das Polymer, nicht aber das Salz lösen. Dadurch ist gewährleistet, dass die porogenen Eigenschaften des Salzes nicht oder nur unwesentlich beeinflusst werden. Aceton, Ethylacetat, Methylenchlorid, Chloroform, Hexafluorisopropanol, chlorierte und fluorierte, aliphatische und aromatische Kohlenwasserstoffe, Tetrahydrofuran, Ethylmethylketon, Diethylketon sowie Gemische davon eignen sind beispielsweise zum Lösen der vorstehend genannten Polymere. Zum Lösen von Poly(glykolsäure), Poly(milchsäure) oder Poly(glykolsäuremilchsäure) und mit Blick auf die medizinische Verwendung eignet sich insbesondere Chloroform.

Gibt man die Polymerlösung und das Polymerpartikel/Salzpartikel-Gemisch zusammen, entsteht zunächst eine rührbarer Brei, der dann unter Entfernung des Lösungsmittel rasch fest wird. Konzentration des Polymers in der Lösung sind zweckmäßigerweise so zu wählen, dass einerseits das Polymer vollständig gelöst ist, andererseits das Lösungsmittel rasch entfernt werden kann ohne die Polymerpartikel in nennenswertem Umfang anzulösen.

Als günstig hat sich ein Gewichtsverhältnis von Polymerpartikeln zu gelöstem Polymer 10:1 bis 1:100, vorteilhafterweise 2:1 bis 1:25 und insbesondere 1:1 bis 1:10 erwiesen.

Was das Gewichtsverhältnis von Polymerpartikeln zu Kochsalzpartikeln angeht, so kann im Rahmen dieser Ausführungsform ein zugunsten von Kochsalz höheres Gewichtsverhältnis von bis zu 1:200, 1:500 oder 1:1000 gewählt werden, wobei das Gewichtsverhältnis von Gesamtpolymer zu Kochsalz nach wie größer als 1:100 ist. Auf diese Art und Weise gelingt es, Porositäten oberhalb von 98 % einzustellen.

Bei dem vorstehend beschriebenen Verfahren dient das Kochsalz als porogenes Material, womit definitionsgemäß ein festes oder zumindest halbfestes Material gemeint ist, das mit dem matrixbildenden Polymer zunächst zu einem Gemisch vereinigt und dann aus dem Gemisch wieder entfernt wird, wodurch Hohlräume (Poren) entstehen. Dazu ist es zweckmäßig, dass das porogene Material in wenigstens einem Lösungsmittel löslich und in wenigstens einem weiteren Lösungsmittel im Wesentlichen unlöslich ist. Im Wesentlichen unlöslich ist ein Material insbesondere dann, wenn es unter den Verarbeitungsbedingungen, d.h. in der Regel bei Temperaturen im Bereich von 18 °C bis 25 °C und unter Normaldruck, zu weniger als 30 Gew.-%, vorzugsweise zu weniger als 20 Gew.-%, insbesondere zu weniger als 10 Gew.-%, beispielsweise zu weniger als 5, 4, 3, 2 und 1 Gew.-% löslich ist.

Struktur und Eigenschaften der resultierenden Matrices werden wesentlich durch das zu ihrer Herstellung verwendete porogene Material bestimmt. Dabei spielen nicht nur die Art des porogenen Materials, sondern vor allem die Korngrößenverteilung der porogenen Partikel eine Rolle. So gilt im Allgemeinen, dass mit zunehmender Korngröße nicht nur die Porengröße, sondern auch die Konnektivität, d.h. das Netzwerk miteinander kommunizierender Hohlräume, zunimmt. Dieses Netzwerk, auch Makrostruktur oder makroporöse Struktur genannt, ist zu unterscheiden von den durch Schäumen erhältlichen Poren, die in der Regel geschlossen sind und daher eine als Makrostruktur oder mikroporös bezeichnete Struktur ausbilden.

Verfahren zur Herstellung einer porösen Matrix auf Basis eines biologisch verträglichen Polymers oder Polymergemisches, dadurch gekennzeichnet, dass man ein Gemisch aus Polymerpartikeln, Partikeln eines porogenen Materials und einer Polymerlösung kompaktiert und anschließend das porogene Material herauslöst.

Diese Verfahren ist grundsätzlich nicht auf die zuvor beschriebenen Merkmale beschränkt. So kann das Polymer ausgewählt sein unter Polyanhydriden, Poly(orthoestern), Poly(a-hydroxyestern), Poly(esteramiden), Polyamiden, Poly(esterethern), Polycarbonaten, Polyalkylenen, Polyalkylenglykolen, Polyalkylenoxiden, Polyalkylenterephthalaten, Polyvinylalkoholen, Polyvinylethern, Polyvinylestern, Polyvinylhalogeniden, Polyvinylpyrrolidonen, Polysiloxanen, Polystyrolen, Polyurethanen, derivatisierten Cellulosen, (Meth)acrylsäurepolymeren und -copolymeren. Das porogene Material ist Natriumchlorid. Polymer, porogenes Material und das zur Bildung der Lösung verwendete Lösungsmittel sind grundsätzlich so aufeinander abzustimmen, dass die Lösung Polymer in gelöster und Polymerpartikel in fester Form enthält sowie das porogene Material im wesentlichen nicht löst.

Gegenstand der vorliegenden Erfindung sind Implantate, die wenigstens eine der vorstehend beschriebenen Matrices und wenigstens eine Zelle umfassen. Je nach Zweck des Implantats können die Zellen dabei insbesondere ausgewählt sein unter Leberzellen, Pankreaszellen, Fettzellen, Darmzellen, Hautzellen, Gefäßzellen, Nervenzellen, Muskelzellen, Schilddrüsenzellen und Zahnwurzelzellen. Besondere Ausführungsformen erfindungsgemäßer Implantate betreffen Leberzellen und Pankreaszellen.

Ein weiterer Gegenstand der vorliegenden Erfindung sind Implantate, die wenigstens eine Matrix auf Basis eines biologisch verträglichen Polymers und Zellen wenigstens zweier Zelltypen umfassen, wobei die Zellen des ersten Zelltyps Hepatozyten und die Zellen des zweiten Zelltyps Langerhans'sche Inselzellen sind. Dieser Gegenstand ist nicht auf die vorstehend beschriebenen Matrices, d.h. Implantate auf Basis der Matrices, beschränkt.

Je nach Zweck des Implantats, d.h. insbesondere der zu erfüllenden Funktion, sind bestimmte Verhältnisse von Hepatozyten zu Langerhans'schen Inselzellen von Vorteil. So betrifft eine Ausführungsform der Erfindung Implantate, die nach Implantation die endokrinen Eigenschaften eines äquivalenten Pankreasorgans zeigen. Hierfür hat sich ein Verhältnis von Hepatozyten zu Langerhans'schen Inselzellen von 10⁶: 3000 als vorteilhaft erwiesen. Eine weitere Ausführungsform der Erfindung betrifft Implantate, die nach Implantation Stoffwechselfunktionen einer Leber vollziehen. Hierfür hat sich ein Verhältnis von Hepatozyten zu Langerhans'schen Inselzellen von 10⁶: 3-200, vorteilhafterweise von 10⁶: 10-100, insbesondere von 10⁶: 20-80 und besonders bevorzugt von 10⁶: 35-45 als zweckmäßig erwiesen.

Es sei angemerkt, dass derartige Implantate in der Regel neben Hepatozyten und Langerhans'schen Inselzellen weitere Zellen beinhalten, nämlich insbesondere weitere Leber- und Pankreaszellen, die bei der Zellisolation mit anfallen.

Die zur Besiedlung poröser Matrices zu verwendenden Zellen oder Zellgemische können in an sich bekannter Weise gewonnen werden. Im Sinne eines autologen Implantats stammen die Zellen vorzugsweise aus dem Individuum, dem das Implantat eingesetzt werden soll. So wird dem Individuum in der Regel geeignetes Gewebe, beispielsweise ein Stück Leber oder Pankreas, entnommen und in geeigneterweise für die Beimpfung und *in vitro*-Kultur der Matrix vorbereitet. Hierbei ist es von Bedeutung, dass die Zellen eine möglichst hohe Vitalitätsrate aufweisen.

Gewinnt man Leberzellen aus Lebergewebe, ist zu beachten, dass die Leberzellen insbesondere im Falle einer Leberzirrhose von einer starken Bindegewebsschicht umgeben sind. Um die Leberzellen mit einem möglichst hohen Anteil vitaler Zellen isolieren zu können, werden erfindungsgemäß Lösungen bestimmter Zusammensetzung verwendet.

Eine NaCl, KCl und HEPES enthaltende, wässrige Zusammensetzung A mit einem pH-Wert von etwa 7,4 ist nützlich für die Verwendung zum Perfundieren eines Leber- oder Pankreasstücks. Insbesondere enthalten 1000 ml dieser Lösung etwa 8,3 g NaCl, 0,5 g KCl und 2,38 g HEPES. Das Perfundieren erfolgt vorzugsweise bei einer Temperatur von etwa 37 °C und einer Flussrate von etwa 30 ml/min. Wenige Minuten, insbesondere etwa 5 bis 120 Minuten, beispielsweise etwa 7 Minuten, reichen aus, um bei der vorstehend genannten Flussrate das Gewebestück ausreichend zu perfundieren.

Alternativ dazu kann man auch eine Ethylenglykoltetraessigsäure (EGTA) enthaltende, wässrige Zusammensetzung A' zum Perfundieren eines Leber- oder Pankreasstücks verwenden.

Eine wässrige Zusammensetzung B mit einem pH-Wert von etwa 7,3 bis 7,4, vorzugsweise etwa 7,35, welche NaCl, KCl, HEPES, CaCl₂, Collagenase und Trypsininhibitor enthält, ist nützlich für die Verwendung zum Perfundieren eines Leber- oder Pankreasstücks. Vorzugweise enthalten 1000 ml der Lösung 8,3 g NaCl, 0,5 g KCl, 2,38 g HEPES, 0,7 g CaCl₂ x 2 H₂O, 500 mg Collagenase H und 7,5 mg Trypsininhibitor. Auch in diesem Fall hat sich das Perfundieren bei etwa 37 °C und einer Flussrate von etwa 30 ml/min als zweckmäßig erwiesen. Wenige Minuten, insbesondere etwa 5 bis 10 Minuten, beispielsweise etwa 6 bis 7 Minuten, reichen aus, um das Gewebestück hinreichend zu perfundieren.

Alternativ dazu kann man auch eine wässrige Zusammensetzung B' zum Perfundieren eines Leber- oder Pankreasstücks verwenden, welche Kollagenase und Hyaluronidase enthält. Vorzugsweise enthalten 1000 ml der Lösung 5 bis 10 U/ml Kollagenase und 5 bis 10 U/ml Hyaluronidase.

Es ist für die Vitalität der zu gewinnenden Zellen von Vorteil, wenn das Gewebestück zunächst mit Zusammensetzung A und anschließend mit Zusammensetzung B behandelt wird. Alternativ dazu kann zunächst eine Zusammensetzung A' und dann eine Zusammensetzung B' verwendet werden.

Im Anschluss an die Perfusion kann das Gewebestück dann frei präpariert werden und in einem geeigneten Medium, beispielsweise Williams-Medium E, vorsichtig aufgeschüttelt werden. Enthält die resultierende Zellsuspension noch gröbere Zelltrümmer, können diese in an sich bekannter Weise entfernt werden, beispielsweise indem man die Zellsuspension über ein Nylonnetz (200 um) filtriert. Die Zellen des Filtrats können dann vorsichtig pelletiert werden, wobei sich eine dreiminütige Zentrifugation bei 50 g und 4 °C als vorteilhaft erwiesen hat.

Die Auftragung der gewonnenen Zellen auf die Matrices erfolgt in an sich bekannter Weise. In der Regel werden die Zellen als zellhaltige Lösung auf die Matrix aufgetragen und anschließend - üblicherweise unter Zellkulturbedingungen -inkubiert, bis Zellen an der Matrix anhaften. Werden mehr als ein Zelltyp, beispielsweise Hepatocyten und Langerhans'sche Inselzellen, auf eine Matrix aufgetragen, können die verschiedenen Zelltypen im Prinzip gemeinsam oder aber nacheinander aufgetragen werden. Einer besonderen Ausführungsform zufolge trägt man zunächst Langerhans'schen Inselzellen und anschließend Hepatocyten auf, wobei man nach dem Auftragen jeweils inkubiert, bis zumindest ein Teil der Zellen an der Matrix anhaftet.

Erfindungsgemäße Implantate weisen entscheidende Vorteile auf. So ermöglichen die inneren Dimensionen der Matrices eine effiziente Besiedlung mit Zellen. Die Matrices sind einerseits frei verformbar und bieten andererseits ausreichend Stabilität und Rigidität, um die chirurgische Implantationsprozedur zu überstehen und den am Implantationsort einwirkenden mechanischen Kräften zu widerstehen. Der nach der Implantation einsetzende, initiale Zelluntergang ist begrenzt und implantiertes Gewebe kann nach kurzer Zeit die beabsichtigte Funktion aufnehmen. Kurz nach der Implantation kommt es zur Gefäßeinsprossung bzw. zur Einsprossung von gefäßreichem Granulationsgewebe und auch von Nervengewebe. Die porösen Matrices können hergestellt werden, ohne physiologisch bedenkliche Lösungsmittel, beispielsweise Formaldehyd, verwenden zu müssen, so dass kein spezielles Verfahren zur Elimination der Lösungsmittel erforderlich ist und die Gefahr verbleibender Restmengen dieser Lösungsmittel nicht besteht.

Erfindungsgemäße Implantate weisen vielfältige Verwendungsmöglichkeiten auf. Hiervon sind insbesondere Verwendungen im medizinischen Bereich zu nennen. Ein weiterer Gegenstand der vorliegenden Erfindung sind daher die erfindungsgemäßen Implantate zur therapeutischen Verwendung.

Eine besondere Verwendung in diesem Bereich ist die des Aufbaus von Gewebe (Tissue Engineering). Dabei dienen die porösen Matrices quasi als Gerüst (Scaffold), in das Zellen einwandern und/oder sich anheften.

Dazu kann man die Matrices beispielsweise in vitro mit den gewünschten Zellen beimpfen, z.B. mit einer zellhaltigen Lösung versetzen und inkubieren, bis Zellen sich an die Matrix angeheftet haben. Eine solche Matrix mit daran anhaftenden Zellen (hier mit Implantat bezeichnet) kann dann weiteren Verfahrensmaßnahmen, beispielsweise weiterer Kultivierung, gegebenenfalls unter Einwirkung von Wirkstoffen, z.B. zur weiteren Expansion der Zellen oder zur Modulierung ihrer Eigenschaften, unterzogen werden, und/oder bis zur Implantation in geeigneter Weise, beispielsweise auf Eis oder in einem Bioflussreaktor unter Standardbedingungen, aufbewahrt werden. Im Rahmen dieser Verwendung ist es von Vorteil, die zur Implantation bestimmten Zellen zunächst in vitro isolieren und gegebenenfalls auch expandieren zu können. Insbesondere wird dadurch das Aufbringen verschiedener Zelltypen, wie die oben beschriebenen Hepatozyten zusammen mit Langerhans'schen Inselzellen, auf eine Matrix ermöglicht.

Anstelle einer in vitro Beimpfung besteht eine weitere Möglichkeit darin, die Matrix (ohne vorherige Zellanheftung) zu implantieren mit dem Ziel, zur Geweberegenerierung befähigte Vorläuferzellen zu veranlassen, in ein geschädigtes Gewebe einzuwandern und dort verloren gegangenes Gewebe zu regenerieren. Die Matrix muss dazu so gestaltet sein, dass erwünschte Zellen, nicht aber unerwünschte Zellen, in die Matrix einwandern können. Eine derartige Verwendung wird im Allgemeinen als geführte Geweberegenerierung (GTR für "Guided Tissue Regeneration") bezeichnet.

Ein erfindungsgemäßes Implantat kann daher zur Verwendung bei der Behandlung des menschlichen oder tierischen Körpers dienen. Dazu werden eine oder mehrere Matrices bzw. ein oder mehrere Implantate dem zu behandelnden Körper im Wege eines chirurgischen Eingriffs eingesetzt. Beinhaltet das Implantat Zellen mit Organfunktion bzw. sollen in die Matrix Zellen mit Organfunktion einwandern, wir es beispielsweise bei Hepatozyten oder Langerhans'schen Inselzellen der Fall ist, können die Matrices oder Implantate beispielsweise in das Mesenterium, subkutane Gewebe, Retroperitoneum, den properitonealen Raum oder den intramuskulären Raum des zu behandelnden Individuums implantiert werden.

Mit den erfindungsgemäßen Implantaten können im Prinzip sämtliche Individuen behandelt werden, die einen entsprechenden Gewebeersatz benötigen. Dies sind in der Regel Individuen, die an einer bestimmten Störung oder Erkrankung leiden, in deren Verlauf es zum Verlust von funktionsfähigem Gewebe kommt. Dies kann unter Umständen ganze Organe, beispielsweise die Leber oder das Pankreas betreffen. Die Implantate der vorliegenden Erfindung sind insbesondere brauchbar zur Behandlung von Erkrankungen, die zu chronischem Leber- oder Pankreasversagen führen. Hierzu gehören beispielsweise chronische Hepatitis und biliäre Zirrhose bei Erwachsenen sowie biliäre Atresie und angeborene metabolische Defekte bei Kindern. Auch bei Leberkarzinomen kann eine Lebertransplantation angezeigt sein. Eine Pankreastransplantation hingegen ist insbesondere angezeigt bei allen Formen eines Diabetes mellitus, insbesondere einem vom Typ I oder II.

Gegenstand der vorliegenden Erfindung ist daher auch ein erfindungsgemäßes Implantat zur Verwendung bei der Transplantation eines Individuums und dabei insbesondere zur Behandlung eines Individuums, das an einem zumindest partiellen Verlust funktionsfähigen Gewebes leidet, welches durch das Transplantat ersetzt werden soll.

Die folgenden Beispiele sollen die Erfindung veranschaulichen, ohne sie in ihrem Umfang einzuschränken.

### Beispiel 1 Herstellung der Matrix

### a) Ohne Polymerlösung

Polymer-Pellets (Resomer® RG 858, erhältlich von der Firma Boehringer, Ingelheim) werden in Flüssigstickstoff gefroren und in gefrorenem Zustand geschreddert (Schlagwerk der Firma Däschle; 12000 U/min 2 min). Die geschredderten Polymerpartikel werden gesiebt. Partikel mit einer Größe von 108 µm bis 250 µm werden für die Matrixherstellung eingesetzt. Dabei besitzen 16 Gew.-% des eingesetzten Polymers eine Partikelgröße zwischen 108 µm und 140 µm, 22 Gew.-% des eingesetzten Polymers eine Partikelgröße zwischen 145 µm und 180 µm, 46 Gew.-% des eingesetzten Polymers eine Partikelgröße zwischen 185 µm und 220 µm, und 16 Gew.-% des eingesetzten Polymers eine Partikelgröße zwischen 225 µm und 250 µm. Kochsalz wird gesiebt und Kochsalzpartikel mit einer Korngröße von 250 µm bis 425 µm werden für die Matrixherstellung verwendet. Dabei besitzen 25 Gew.-% des eingesetzten Salzes eine Partikelgröße zwischen 250 µm und 320 µm, 44 Gew.-% des eingesetzten Salzes eine Partikelgröße zwischen 330 µm und 380 µm, und 31 Gew.-% des eingesetzten Salzes eine Partikelgröße zwischen 390 µm und 425 µm. 760 mg Kochsalzpartikel und 40 mg Polymerpartikel werden miteinander vermischt. Das Gemisch wird in eine Stanzform eingebracht und mit einer Hydraulikpresse bei einem Stempeldruck von 1000 psi 1 Minute gepresst. Anschließend werden die Matrixrohlinge auf einen Teflonteller gelegt und 24 Stunden in einer CO-Atmosphäre (850 psi) begast.

Dann werden die Rohlinge 24 Stunden gewässert, um die eingeschlossenen Salzkörner aufzulösen. Schließlich werden die Matrices 12 Stunden auf Saugpapier getrocknet.

Die resultierende Polymermatrix weist eine Porosität von 95 +/- 2% und eine mittels Rasterelektronenmikroskopie bestimmte, definierte Porengröße von 250 µm +/-120 µm auf.

### b) Mit Polymerlösung

Kochsalz (analytisch rein) wird gemahlen (Schlagwerk der Firma Däschle; 12000 U/min 2 min) und anschließend gesiebt, und Kochsalzpartikel mit einer Korngröße von 108 bis 425 µm werden für die Matrixherstellung verwendet. Dabei besitzen 8 % des eingesetzten Salzes eine Partikelgröße zwischen 108 µm und 140 µm, 6 Gew.-% des eingesetzten Salzes eine Partikelgröße zwischen 145 µm und 180 µm, 12 Gew.-% des eingesetzten Salzes eine Partikelgröße zwischen 185 µm und 220 µm, 6 Gew.-% des eingesetzten Salzes eine Partikelgröße zwischen 225 µm und 250 µm, 25 Gew.-% des eingesetzten Salzes eine Partikelgröße zwischen 250 µm und 320 µm, 26 Gew.-% des eingesetzten Salzes eine Partikelgröße zwischen 330 µm und 380 µm, und 17 Gew.-% des eingesetzten Salzes eine Partikelgröße zwischen 390 µm und 425 µm. 96 g Kochsalzpartikel werden mit 1 g der in Beispiel 1 a) beschriebenen Polymerpartikel vermischt und anschließend mit 100 ml einer Chloroformlösung, die 4 g des Polymers gelöst enthält, versetzt. Das so erhaltene Gemisch wird bei 45 °C bis 65 °C erwärmt, wodurch das Chloroform innerhalb von etwa 25 Minuten verdampft. Das verbleibende Salz-Polymer-Gemisch wird dann mit einer Hydraulikpresse bei einem Stempeldruck von 1000 psi eine Minute gepresst und anschließend 24 Stunden gewässert, um die eingeschlossenen Salzkörner aufzulösen. Anschließend wird die Matrix, wie oben beschrieben, begast und schließlich 12 Stunden auf Saugpapier getrocknet.

Die resultierende Polymermatrix weist eine Porosität von 96 % auf.

Vermischt man 98,5 g Salzpartikel mit 0,5 g Polymerpartikel und versetzt das Gemisch mit 100 ml einer Chloroform-Lösung, die 1 g Polymer enthält, erhält man eine Matrix mit einer Porosität von 99 %.

Vermischt man 99,2 g Salzpartikel mit 0,1 g Polymerpartikel und versetzt dieses Gemisch mit 100 ml einer Chloroform-Lösung, die etwa 0,9 g Polymer enthält, erhält man eine Polymermatrix mit einer Porosität von 99 %.

### Beispiel 2

### a) Beschichtung der Matrix mit Fibronectin

Die Matrix aus Beispiel 1 wird in eine 3 µg/ml Fibronectin aus Humanplasma (Sigma) enthaltende Carbonatpufferlösung mit einem pH-Wert von 9,4 getaucht. Nach etwa 60 s wird die Matrix aus der Lösung entnommen, lyophilisiert und γ-sterilisiert.

### Beispiel 3

### Zellisolation

Dem zu transplantierenden Individuum wird in an sich bekannter Weise ein Leberstück entnommen. Das entnommene Leberstück wird zunächst 7 Minuten bei einer Flussrate von 30 ml/min und 37 °C mit einer Lösung (8,3 g NaCl; 0,5 g KCl; 2,38 g HEPES; ad 1000 ml destilliertes Wasser; pH-Wert 7,4) perfundiert. Anschließend wird das Leberstück weitere 6 bis 7 min bei einer Flussrate von 30 ml/min und 37 °C mit einer Collagenase-Trypsin-Inhibitor-Lösung (8,3 g NaCl; 0,5 g KCl; 2,38 g HEPES; 0,7 g CaCl₂ x 2 H₂O; 500 mg Collagenase (Collagenase H, Boehringer Mannheim, Mannheim, Deutschland); 7,5 mg Trypsin-Inhibitor (ICN, Eschwege, Deutschland); ad 1000 ml destilliertes Wasser; pH-Wert 7,35) perfundiert. Nach Beendigung der Perfusion wurde das Leberstück frei präpariert und in Williams-Medium E vorsichtig aufgeschüttelt. Die Zellsuspension wird filtriert (Nylonnetz; 200 um) und anschließend mit Williams-Medium E gewaschen. Anschließend werden die Zellen bei 3 min bei 50 g und 4 °C zentrifugiert. Die mit Tryptan-Blau bestimmte Vitalität der Zellen beträgt 95 %.

In gleicher Weise werden Langerhans'sche Inselzellen aus einem Pankreasstück isoliert.

### Beispiel 4

### Zellbesiedlung

Die in Beispiel 2 beschichteten Matrices werden im ersten Schritt mit Langerhans'schen Inselzellen inkubiert, welche gemäß Beispiel 3 isoliert wurden.

Dazu wurden 3000 Inselzellen pro ml in einem Lösungsgemisch aus M199 und FKS (Volumenverhältnis von 19:1) suspendiert. Die Zellzahl wird bestimmt, indem man sie unter einem inversen Olympus-Mikroskop in einem 0,25-mm-Zählrohr ausgezählt. Dann werden 8 ml bis 10 ml dieser Lösung mit einer Pipette auf die Matrix aufgebracht. Die überschüssige Lösung, welche nicht in der Matrix verbleibt, wird verworfen. Die so behandelte Matrix wird anschließend zur Anheftung der Zellen 4 Stunden in den Zellkulturbrutschrank gestellt. Anschließend wird eine Lösung aus Williams Medium E, die pro ml eine nicht gereinigte Leberzellsuspension mit etwa 5,0 x 10⁷ vitalen Hepatocyten und etwa 1,0 x 10⁶ nichtperenchymatösen Leberzellen enthält, auf die Matrix aufgebracht. Es werden 8 ml bis 12 ml Lösung mit einer Pipette aufgetragen; die nicht von der Matrix aufgenommene überschüssige Lösung wird verworfen. Die Matrix kann bis zur Implantation etwa 1,5 Stunden auf Eis gehalten werden. Ist eine Implantation zu einem späteren Zeitpunkt vorgesehen, kann die Matrix in einem Bioflussreaktor unter Standardbedingungen bis zu 5 Tagen aufbewahrt werden.

### Beispiel 5

### Sekretorische Aktivität und Proliferationsrate der Hepatozyten

Lewis-Ratten wurden mit zellbesiedelten Matrices nach Beispiel 4 transplantiert. Die Transplantate wurden den Tieren zu verschiedenen Zeitpunkten wieder entnommen und morphometrisch untersucht. Die Zellzahl der die Form einer kreisrunden Scheibe mit einem Durchmesser von 15 mm und einer Dicke von 2 mm aufweisenden Transplantate betrug 1, 6 und 12 Monate nach Transplantation 94 x 10³, 140 x 10³ bzw. 146 x 10³ Zellen. Hepatozyten des einen Monat nach Transplantation entnommenen Transplantats weisen eine normale Albumin-Expression auf. In allen Präparaten werden proliferierende Hepatozyten gefunden, ohne dass eine pathologisch erhöhte Proliferationsrate vorliegt. Im Vergleich zu Leberstandardpräparaten weisen die erfindungsgemäß transplantierten Hepatozyten einen um den Faktor 3 erhöhten Einbau von BrdU auf.

### Beispiel 6

### Vaskularisierung

Die weitere Untersuchung der in Beispiel 4 beschriebenen Matrices ergibt, dass diese bereits einen Monat nach Implantation hervorragend vaskularisiert sind. Die Blutgefäße reichen makroskopisch bis zur Matrix und die transplantierten Hepatozyten und Langerhans'sche Inselzellen bekommen durch eine ausreichende Kapillarisierung Kontakt zum kardiovaskulären System des Transplantatempfängers.

Es ist weiterhin festzustellen, dass die cotransplantierten Langerhans'sche Inselzellen beim Empfänger keine Hypoglykämie verursachen. Die endokrine Sekretionsleistung dieser Zellen sowie der empfängereigenen Inselzellen wird vermutlich durch einen Rückkopplungsmechanismus geregelt.

### Beispiel 7

### Leberfunktionsübernahme

Gunn-Ratten gelten als Tiermodell für das menschliche Crigler-Najar-Syndrom, da ihre Leber infolge eines spezifischen angeborenen metabolischen Enzymdefekts nicht ausreichend Bilirubin konjugieren kann. Als Folge führen toxische Blutplasmaspiegel unkonjugierten Bilirubins über zahlreiche Folgeschäden zum Tod.

Drei Gunn-Ratten werden mit einer zellbesiedelten Matrix gemäß Beispiel 4 transplantiert. Die Matrix besitzt eine Außenfläche von insgesamt 10 cm².

Bereits vier Wochen nach Transplantation sinkt der Bilirubinspiegel der Versuchstiere. Bilirubin wird nunmehr konjugiert. Das konjugierte Bilirubin kann in allen drei Fällen in den Gallengängen der noch vorhandenen Leber mit Hilfe einer Gallengangsonde nachgewiesen werden. Somit gelangt das in der Matrix konjugierte Bilirubin hämatogen in die Leber und kann dort über das Gallengangsystem ausgeschieden werden.

### Beispiel 8

### Humane Patienten

Ein Patient mit einer ausgeprägten Leberzirrhose wird mit zellbesiedelten Matrices gemäß Beispiel 4 in die Bauchhöhle transplantiert. Die folgende Tabelle 1 fasst die Laborbefunde des Patienten vor der Transplantation zusammen.

**Tabelle 1**

| Parameter | Patient 1 |
|---|---|
| GOT | 27 |
| GPT | 35 |
| gGt | 89 |
| CHE | 2421 |
| Serumalbumin | 24,1 |

Patient 1 (Ethyltoxische Leberzirrhose, zuvor mehrfach dekompensiert, jetzt nicht aktiv) erhielt 4 Matrices (je 124 mm x 45 mm x 5 mm).

Die folgende Tabelle 2 fasst die Leberwerte 3, 10 bzw. 20 Wochen nach Transplantation zusammen.

**Tabelle 2**

| | Patient 1 | | |
|---|---|---|---|
| | 3 | 10 | 20 |
| GOT | 22 | 10 | 11 |
| GPT | 28 | 9 | 28 |
| gGt | 71 | 10 | 9 |
| Serumalbumin | 28,6 | 42 | 44 |
| CHE | 2652 | 4400 | 4600 |

## Patentansprüche

1. Autologes Implantat zur Verwendung bei der Behandlung eines Individuums, das einer Leber- oder Pankreastransplantation bedarf, wobei das autologe Implantat erhältlich ist durch ein Verfahren, welches das Beimpfen einer porösen Matrix auf Basis eines biologisch verträglichen Polymers oder Polymergemisches mit vitalen Leber- oder Pankreaszellen des Wesens umfasst, wobei die poröse Matrix erhältlich ist, indem man ein Gemisch aus Polymerpartikeln mit einer Korngröße im Bereich von 20 bis 950 µm und Kochsalzpartikeln mit einer Korngröße im Bereich von 90 bis 670 µm kompaktiert und anschließend das Kochsalz herauslöst, und wobei die poröse Matrix Poren mit einer Größe von 130 µm oder weniger und Poren mit einer Größe von 370 µm oder mehr aufweist und der Porositätsgrad die zahlenmäßige Angabe in % zum Anteil des Porenvolumens am Gesamtvolumen der Matrix ist und 93 bis 98 % beträgt und wobei die Matrix folgende Porengrößenverteilung aufweist: 0,5 % bis 6 % Poren mit einem mittleren Durchmesser im Bereich von 70 bis 100 µm; 2 % bis 8 % Poren mit einem mittleren Durchmesser im Bereich von 101 bis 115 µm; 2 % bis 8 % Poren mit einem mittleren Durchmesser im Bereich von 116 bis 130 µm; 1 % bis 7 % Poren mit einem mittleren Durchmesser im Bereich von 131 bis 300 µm; 11 % bis 23 % Poren mit einem mittleren Durchmesser im Bereich von 301 bis 330 µm; 4 % bis 10 % Poren mit einem mittleren Durchmesser im Bereich von 331 bis 360 µm; 5 % bis 17 % Poren mit einem mittleren Durchmesser im Bereich von 361 bis 390 µm; 7 % bis 19 % Poren mit einem mittleren Durchmesser im Bereich von 391 bis 420 µm; 3 % bis 9 % Poren mit einem mittleren Durchmesser im Bereich von 421 bis 450 µm; 12 % bis 24 % Poren mit einem mittleren Durchmesser im Bereich von 451 bis 480 µm; und 5 % bis 17 % Poren mit einem mittleren Durchmesser im Bereich von 481 bis 510 µm, wobei die Porengrößen und die Porengrößenverteilung durch Rasterelektronenmikroskopie bestimmt werden.

2. Implantat zur Verwendung nach Anspruch 1, **dadurch gekennzeichnet, dass** das biologisch abbaubare Polymer Poly(glycolsäure-milchsäure) mit einem Milchsäure-Anteil von 85 mol-% und einem Glycolsäure-Anteil von 15 mol-% ist.

3. Implantat zur Verwendung nach Anspruch 1 oder 2, **dadurch gekennzeichnet, dass** das Gewichtsverhältnis von Polymerpartikeln zu Kochsalzpartikeln 1:100 bis 1:10 beträgt.

4. Implantat zur Verwendung nach Anspruch 1 oder 2, **dadurch gekennzeichnet, dass** das Gewichtsverhältnis von Polymerpartikeln zu Kochsalzpartikeln 1:50 bis 1:15 beträgt.

5. Implantat zur Verwendung nach Anspruch 1 oder 2, **dadurch gekennzeichnet, dass** das Gewichtsverhältnis von Polymerpartikeln zu Kochsalzpartikeln 1:20 bis 1:18 beträgt.

6. Implantat zur Verwendung nach einem der Ansprüche 2 bis 5, **dadurch gekennzeichnet, dass** man vor dem Kompaktieren dem Gemisch aus Polymerpartikeln und Kochsalzpartikeln eine Polymerlösung zusetzt und das Lösungsmittel entfernt.

7. Implantat zur Verwendung nach Anspruch 6, **dadurch gekennzeichnet, dass** das Polymer Poly(glykolsäure), Poly(milchsäure) oder Poly(glykolsäuremilchsäure) und das Lösungsmittel Chloroform ist.

8. Implantat zur Verwendung nach Anspruch 6 oder 7, **dadurch gekennzeichnet, dass** das Gewichtsverhältnis von Polymerpartikeln zu gelöstem Polymer unter den folgenden Bereichen ausgewählt ist: 10:1 bis 1:100, 2:1 bis 1:25 und 1:1 bis 1:10.

9. Implantat zur Verwendung nach einem der Ansprüche 6 bis 8, **dadurch gekennzeichnet, dass** die Polymerlösung Polymer in gelöster und Polymerpartikel in fester Form enthält.

10. Implantat zur Verwendung nach einem der Ansprüche 6 bis 9, **dadurch gekennzeichnet, dass** die Polymerlösung das Kochsalz nicht löst.

11. Implantat zur Verwendung nach einem der Ansprüche 1 bis 10, **dadurch gekennzeichnet, dass** die Matrix Zellen wenigstens zweier Zelltypen umfasst, wobei die Zellen des ersten Zelltyps Hepatozyten und die Zellen des zweiten Zelltyps Langerhans'sche Inselzellen sind.

12. Implantat zur Verwendung nach Anspruch 11, **dadurch gekennzeichnet, dass** das Verhältnis von Hepatozyten zu Langerhans'schen Inselzellen 10⁶ : 3000 beträgt.

13. Implantat zur Verwendung nach Anspruch 11, **dadurch gekennzeichnet, dass** das Verhältnis von Hepatozyten zu Langerhans'schen Inselzellen unter den folgenden Bereichen ausgewählt ist: 10⁶ : 3-200, 10⁶ : 10-100, 10⁶ : 20-80 und 10⁶ : 35-45.

## Claims

1. Autologous implant for use in the treatment of an individual requiring a liver or pancreas transplant, wherein the autologous implant is obtainable by a method comprising the inoculation of a porous matrix based on a biologically compatible polymer or polymer mixture with viable liver or pancreas cells from the living being, wherein the porous matrix is obtainable by compacting a mixture of polymer particles having a particle size within the range from 20 to 950 µm and sodium chloride particles having a particle size within the range from 90 to 670 µm and then dissolving out the sodium chloride, and wherein the porous matrix comprises pores of a size of 130 µm or less and pores of a size of 370 µm or more and the degree of porosity is the numerical value in % relating to the pore-volume-based proportion of the total volume of the matrix and is 93 to 98%, and wherein the matrix has the following pore size distribution: 0.5% to 6% of pores having a mean diameter within the range from 70 to 100 µm; 2% to 8% of pores having a mean diameter within the range from 101 to 115 µm; 2% to 8% of pores having a mean diameter within the range from 116 to 130 µm; 1% to 7% of pores having a mean diameter within the range from 131 to 300 µm; 11% to 23% of pores having a mean diameter within the range from 301 to 330 µm; 4% to 10% of pores having a mean diameter within the range from 331 to 360 µm; 5% to 17% of pores having a mean diameter within the range from 361 to 390 µm; 7% to 19% of pores having a mean diameter within the range from 391 to 420 µm; 3% to 9% of pores having a mean diameter within the range from 421 to 450 µm; 12% to 24% of pores having a mean diameter within the range from 451 to 480 µm; and 5% to 17% of pores having a mean diameter within the range from 481 to 510 µm, wherein the pore sizes and the pore size distribution are determined by scanning electron microscopy.

2. Implant for use according to Claim 1, **characterized in that** the biodegradable polymer is poly(glycolic acid-lactic acid) having a lactic acid fraction of 85 mol% and a glycolic acid fraction of 15 mol%.

3. Implant for use according to Claim 1 or 2, **characterized in that** the weight ratio of polymer particles to sodium chloride particles is 1:100 to 1:10.

4. Implant for use according to Claim 1 or 2, **characterized in that** the weight ratio of polymer particles to sodium chloride particles is 1:50 to 1:15.

5. Implant for use according to Claim 1 or 2, **characterized in that** the weight ratio of polymer particles to sodium chloride particles is 1:20 to 1:18.

6. Implant for use according to any one of Claims 2 to 5, **characterized in that**, prior to compacting the mixture of polymer particles and sodium chloride particles, a polymer solution is added and the solvent is removed.

7. Implant for use according to Claim 6, **characterized in that** the polymer is poly(glycolic acid), poly(lactic acid) or poly(glycolic acid-lactic acid) and the solvent is chloroform.

8. Implant for use according to Claim 6 or 7, **characterized in that** the weight ratio of polymer particles to dissolved polymer is selected from the following ranges: 10:1 to 1:100, 2:1 to 1:25 and 1:1 to 1:10.

9. Implant for use according to any one of Claims 6 to 8, **characterized in that** the polymer solution contains polymer in dissolved form and polymer particles in solid form.

10. Implant for use according to any one of Claims 6 to 9, **characterized in that** the polymer solution does not dissolve the sodium chloride.

11. Implant for use according to any one of Claims 1 to 10, **characterized in that** the matrix comprises cells of at least two cell types, the cells of the first cell type being hepatocytes and the cells of the second cell type being cells of the islets of Langerhans.

12. Implant for use according to Claim 11, **characterized in that** the ratio of hepatocytes to cells of the islets of Langerhans is 10⁶:3000.

13. Implant for use according to Claim 11, **characterized in that** the ratio of hepatocytes to cells of the islets of Langerhans is selected from the following ranges: 10⁶:3-200, 10⁶:10-100, 10⁶:20-80 and 10⁶:35-45.

## Revendications

1. Implant autologue destiné à être utilisé pour le traitement d'un individu qui a besoin d'une transplantation du foie ou du pancréas, l'implant autologue pouvant être obtenu par un procédé qui comprend l'ensemencement d'une matrice poreuse à base d'un polymère ou d'un mélange polymère biologiquement acceptable par des cellules hépatiques ou pancréatiques vitales de l'être, la matrice poreuse pouvant être obtenue en ce qu'on compacte un mélange constitué par des particules polymères présentant une grosseur de particule dans la plage de 20 à 950 µm et des particule de chlorure de sodium présentant une grosseur de particule dans la plage de 90 à 670 µm et on élimine ensuite le chlorure de sodium par dissolution et la matrice poreuse présentant des pores d'une dimension de 130 µm ou moins et des pores d'une dimension de 370 µm ou plus et le degré de porosité étant l'indication numérique en % de la proportion du volume de pores par rapport au volume total de la matrice et valant 93 à 98% et la matrice présentant la répartition suivante des dimensions de pores : 0,5% à 6% de pores présentant un diamètre moyen dans la plage de 70 à 100 µm ; 2% à 8% de pores présentant un diamètre moyen dans la plage de 101 à 115 µm ; 2% à 8% de pores présentant un diamètre moyen dans la plage de 116 à 130 µm ; 1% à 7% de pores présentant un diamètre moyen dans la plage de 131 à 300 µm ; 11% à 23% de pores présentant un diamètre moyen dans la plage de 301 à 330 µm ; 4% à 10% de pores présentant un diamètre moyen dans la plage de 331 à 360 µm ; 5% à 17% de pores présentant un diamètre moyen dans la plage de 361 à 390 µm ; 7% à 19% de pores présentant un diamètre moyen dans la plage de 391 à 420 µm ; 3% à 9% de pores présentant un diamètre moyen dans la plage de 421 à 450 µm ; 12% à 24% de pores présentant un diamètre moyen dans la plage de 451 à 480 µm ; et 5% à 17% de pores présentant un diamètre moyen dans la plage de 481 à 510 µm, les dimensions des pores et la répartition des dimensions des pores étant déterminées par microscopie électronique à balayage.

2. Implant destiné à être utilisé selon la revendication 1, **caractérisé en ce que** le polymère biodégradable est le poly(acide glycolique-acide lactique) présentant une proportion d'acide lactique de 85% en mole et une proportion d'acide glycolique de 15% en mole.

3. Implant destiné à être utilisé selon la revendication 1 ou 2, **caractérisé en ce que** le rapport pondéral des particules polymères aux particules de chlorure de sodium est de 1:100 à 1:10.

4. Implant destiné à être utilisé selon la revendication 1 ou 2, **caractérisé en ce que** le rapport pondéral des particules polymères aux particules de chlorure de sodium est de 1:50 à 1:15.

5. Implant destiné à être utilisé selon la revendication 1 ou 2, **caractérisé en ce que** le rapport pondéral des particules polymères aux particules de chlorure de sodium est de 1:20 à 1:18.

6. Implant destiné à être utilisé selon l'une quelconque des revendications 2 à 5, **caractérisé en ce qu'**on ajoute au mélange de particules polymères et de particules de chlorure de sodium, avant le compactage, une solution de polymère et on élimine le solvant.

7. Implant destiné à être utilisé selon la revendication 6, **caractérisé en ce que** le polymère est le poly(acide glycolique), le poly(acide lactique) ou le poly(acide glycolique-acide lactique) et le solvant est le chloroforme.

8. Implant destiné à être utilisé selon la revendication 6 ou 7, **caractérisé en ce que** le rapport pondéral des particules polymères au polymère dissous est choisi parmi les plages suivantes : 10:1 à 1:100, 2:1 à 1:25 et 1:1 à 1:10.

9. Implant destiné à être utilisé selon l'une quelconque des revendications 6 à 8, **caractérisé en ce que** la solution de polymère contient le polymère sous forme dissoute et les particules polymères sous forme solide.

10. Implant destiné à être utilisé selon l'une quelconque des revendications 6 à 9, **caractérisé en ce que** la solution de polymère ne dissout pas le chlorure de sodium.

11. Implant destiné à être utilisé selon l'une quelconque des revendications 1 à 10, **caractérisé en ce que** la matrice comprend au moins deux types de cellules, les cellules du premier type de cellule étant des hépatocytes et les cellules du deuxième type de cellule étant des cellules d'îlots de Langerhans.

12. Implant destiné à être utilisé selon la revendication 11, **caractérisé en ce que** le rapport des hépatocytes aux cellules d'îlots de Langerhans est de 10⁶:3000.

13. Implant destiné à être utilisé selon la revendication 11, **caractérisé en ce que** le rapport des hépatocytes aux cellules d'îlots de Langerhans est choisi parmi les plages suivantes : 10⁶:3-200, 10⁶:10-100, 10⁶:20-80 et 10⁶:35-45.
